# EUROPEAN PATENT SPECIFICATION

(11) **EP 4 543 319 B1**
(45) Date of publication and mention of the grant of the patent: **22.10.2025**
(21) Application number: 24736101.7
(22) Date of filing: 19.06.2024
(51) Int. Cl.: A61B 17/072

(54) **MODIFIED SURGICAL ADJUNCTS AND STAPLING ASSEMBLIES**
MODIFIZIERTE CHIRURGISCHE ZUSÄTZE UND KLAMMERANORDNUNGEN
AUXILIAIRES CHIRURGICAUX MODIFIÉS ET ENSEMBLES D'AGRAFAGE

(30) Priority: 22.06.2023 US 202363522660 P; 11.10.2023 US 202318485047
(43) Date of publication of application: 30.04.2025
(73) Proprietor: Cilag GmbH International, 6300 Zug (CH)
(72) Inventor: HOPSON, Peyton, Cincinnati, Ohio 45242 (US); FRANZONI, Madelaine, Cincinnati, Ohio 45242 (US); FROST, Brody, Cincinnati, Ohio 45242 (US); MCGIVERON, Omar, Cincinnati, Ohio 45242 (US); HEMBRICK-HOLLOMAN, Vincent, Cincinnati, Ohio 45242 (US); VENDELY, Michael J., Cincinnati, Ohio 45242 (US); SEOW, Christopher Q., Cincinnati, Ohio 45242 (US); SALAZAR, Ruben, Cincinnati, Ohio 45242 (US); BRUNS, David, Cincinnati, Ohio 45242 (US); WARD, Andreas, Cincinnati, Ohio 45242 (US); STRANG, Heather, Cincinnati, Ohio 45242 (US)
(74) Representative: Carpmaels & Ransford LLP
(86) International application number: PCT/IB2024/055990
(87) International publication number: WO 2024/261663

(56) References cited:
- US-A1- 2010 116 868
- US-A1- 2015 351 753
- US-A1- 2020 376 158

## Description

### FIELD

The present invention relates generally to stapling assemblies comprising a surgical adjunct and methods of making a surgical tool.

### BACKGROUND

Surgical staplers are used in surgical procedures to close openings in tissue, blood vessels, ducts, shunts, or other objects or body parts involved in the particular procedure. The openings can be naturally occurring, such as passageways in blood vessels or an internal organ like the stomach, or they can be formed by the surgeon during a surgical procedure, such as by puncturing tissue or blood vessels to form a bypass or an anastomosis, or by cutting tissue during a stapling procedure.

Most staplers have a handle (some of which are directly user operable, others of which are operable by a user via a robotic interface) with an elongate shaft extending from the handle and having a pair of movable opposed jaws formed on an end thereof for holding and forming staples therebetween. The staples are typically contained in a staple cartridge, which can house multiple rows of staples and is often disposed in one of the two jaws for ejection of the staples to the surgical site. In use, the jaws are positioned so that the object to be stapled is disposed between the jaws, and staples are ejected and formed when the jaws are closed, and the device is actuated. Some staplers include a knife configured to travel between rows of staples in the staple cartridge to longitudinally cut and/or open the stapled tissue between the stapled rows.

US 2015/0351753 A1 describes adjunct material and methods of using adjunct material to reinforce a staple line. This adjunct material can be coupled to a jaw of a Surgical stapler, and can be deployed into tissue along with the staples. In some embodiments, the adjunct material can be sized and shaped so that a portion of the material extends laterally outside of the staple line and distributes strain to tissue outside of the staple line. In certain aspects, sealant can be applied to the staple line and to the adjunct material in various ways to further seal the tissue and/or prevent leaks from forming in the tissue. This document discloses in Table 1 exemplary molecular weight ranges, approximate absorption times, and average dimensions of films made from porous polymer scaffold materials.

US 2020/0376158 A1 describes tissue repair laminates containing at least two biodegradable polyurethane foam layers and a polyurethane structural layer. The biodegradable polyurethane is derived from biodegradable polyols. The laminates resist shrinkage under in vivo conditions and possess desirable mechanical properties such as high tensile strength. The laminates find use in, for example, the repair of tissue or muscle wall defects.

### SUMMARY

There is provided a stapling assembly. The stapling assembly may include a deck of a cartridge that includes at least one post extending away from the deck. The at least one post may include a first diameter at a top of the at least one post that is greater than a second diameter at the bottom of the at least one post. The cartridge may include a first film disposed on the deck and at least partially surrounding the at least one post with a gap between the top of the at least one post and the film. The cartridge may also include a surgical adjunct disposed on the first film and at least partially surrounding the at least one post. The surgical adjunct includes a polyurethane foam with a volumetric ratio of the polyurethane foam to the total volume of the surgical adjunct in a range of about 0.125 to about 0.325, wherein the surgical adjunct comprises a film disposed on at least one surface of the polyurethane foam, and wherein the film has a thickness FT of about 0.0076 mm (0.0003 inches) to about 0.254 mm (0.010 inches) and has pore diameters of about 0.0127 mm (0.0005 inches) to about 0.127 mm (0.005 inches).

According to the invention and as defined in claim 1, there is provided a stapling assembly. The stapling assembly includes a deck and a surgical adjunct disposed on deck. The surgical adjunct includes a polyurethane foam with a volumetric ratio of the polyurethane foam to the total volume of the surgical adjunct in a range of about 0.125 to about 0.325, wherein the surgical adjunct comprises a film disposed on at least one surface of the polyurethane foam, and wherein the film has a thickness FT of about 0.0076 mm (0.0003 inches) to about 0.254 mm (0.010 inches) and has pore diameters of about 0.0127 mm (0.0005 inches) to about 0.127 mm (0.005 inches).

A method for making a surgical tool is defined in appended claim 9.

Specific embodiments are set forth in the dependent claims.

### BRIEF DESCRIPTION OF THE DRAWINGS

This invention will be more fully understood from the following detailed description taken in conjunction with the accompanying drawings, in which:
FIG. 1 is a perspective view of one exemplary embodiment of a conventional surgical stapling and severing instrument.
FIG. 2A is a top view of a staple cartridge for use with the surgical stapling and severing instrument of FIG. 1;
FIG. 2B is a side view of the staple cartridge of FIG. 2A;
FIG. 3 is a side view of a staple in an unfired (pre-deployed) configuration that can be disposed within the staple cartridge of the surgical cartridge assembly of FIG. 2A;
FIG. 4 is a perspective view of a knife and firing bar ("E-beam") of the surgical stapling and severing instrument of FIG. 1;
FIG. 5 is a perspective view of a wedge sled of a staple cartridge of the surgical stapling and severing instrument of FIG. 1;
FIG. 6A is a longitudinal cross-sectional view of an exemplary surgical cartridge assembly having a compressible non-fibrous adjunct attached to a top or deck surface of a staple cartridge;
FIG. 6B is a longitudinal cross-sectional view of a surgical end effector having an anvil pivotably coupled to an elongate channel and the surgical cartridge assembly of FIG. 6A disposed within and coupled to the elongate channel, showing the anvil in a closed position without any tissue between the anvil and the adjunct;
FIG. 6C is a perspective view of an exemplary surgical end effector having a channel and a surgical cartridge with a sled and drivers;
FIG. 7 is a partial-schematic illustrating the adjunct of FIGS. 6A-6B in a tissue deployed condition;
FIG. 8A is a perspective view of an exemplary cartridge assembly;
FIG. 8B is a side view of an exemplary adjunct for a cartridge assembly;
FIG. 8C is a top view of an exemplary adjunct for a cartridge assembly;
FIG. 8D is a front view of an exemplary adjunct for a cartridge assembly;
FIG. 8E is a diagram showing an enlarged portion of an exemplary adjunct with a porous structure;
FIG. 9A is a side view of an exemplary surgical adjunct with a film;
FIG. 9B is a side view of an exemplary surgical adjunct with a film;
FIG. 9C shows an exemplary cartridge assembly including a surgical adjunct with a film;
FIG. 10A shows an exemplary cartridge assembly with a post and an adhesive film for attaching a surgical adjunct;
FIG. 10B shows an exemplary cartridge assembly with a post and an adhesive film for attaching a surgical adjunct;
FIG. 10C shows an exemplary cartridge assembly with a post, adhesive film, and surgical adjunct;
FIG. 10D is a top view of showing exemplary stakes, staple slots and a perforated film surrounding the staple slots;
FIG. 10E is a top view showing exemplary staples surrounding by films;
FIG. 11A is a flow chart showing an exemplary method for attaching a film to a polyurethane foam;
FIG. 11B is a flow chart showing an exemplary method of disposing a film on a polyurethane foam;
FIG. 11C is a flow chart showing an exemplary method for attaching a surgical adjunct to a cartridge using a film and posts; and
FIG. 12 is graph showing the glass transition temperature Tg of an exemplary adjunct.

### DETAILED DESCRIPTION

The following detailed description should be read with reference to the drawings, in which like elements in different drawings are identically numbered. The drawings, which are not necessarily to scale, depict selected embodiments and are not intended to limit the scope of the invention. The detailed description illustrates by way of example, not by way of limitation, the principles of the invention. This description will clearly enable one skilled in the art to make and use the invention, and describes several embodiments, adaptations, variations, alternatives and uses of the invention, including what is presently believed to be the best mode of carrying out the invention.

As used herein, the terms "about" or "approximately" for any numerical values or ranges indicate a suitable dimensional tolerance that allows the part or collection of components to function for its intended purpose as described herein. More specifically, "about" or "approximately" may refer to the range of values ±10% of the recited value, e.g., "about 90%" may refer to the range of values from 81% to 99%.

Surgical stapling assemblies and methods for manufacturing and using the same are provided. In general, a surgical stapling assembly can include a staple cartridge having staples disposed therein and an adjunct configured to be releasably retained on the staple cartridge. As discussed herein, the various adjuncts provided can be configured to compensate for variations in tissue properties, such as variations in tissue thickness, and/or to promote tissue ingrowth when the adjuncts are stapled to tissue.

An exemplary stapling assembly can include a variety of features to facilitate application of a surgical staple, as described herein and illustrated in the drawings. However, a person skilled in the art will appreciate that the stapling assembly can include only some of these features and/or it can include a variety of other features known in the art. The stapling assemblies described herein are merely intended to represent certain exemplary examples. Moreover, while the adjuncts are described in connection with surgical staple cartridge assemblies, the adjuncts can be used in connection with staple reloads that are not cartridge based or any type of surgical instrument.

The use of absorbable stapling adjuncts or other dampening implants (e.g., ligament anchor surgery, tendon repair) requires a balance between the mechanical strength of the implant/adjunct, chemistry (e.g., absorbable), and deployment requirements. The strength to retain staples, sutures, screws, etc., may conflict with the requirements for endoscopic deployment (e.g., compression through trocar). As the implant strength increases, there is an increased force required to compress the implant for insertion. This may exceed the limits of the endoscopic instrument (e.g., surgical stapling and severing device 100) requiring compromises to the implant performance. While this may be mitigated through phase change materials (e.g., water swell, glass to rubber transitions), this further limit applicable chemistries given the need to have an absorbable implant or adjunct.

A facile method for improving the strength requires to retain stapes, sutures, screws, etc., without compromising the bulk mechanical properties of the implant is the inclusion of a backing material or film. Given the potential need for increase mechanical strength, the backing material may be a different material from the surgical adjunct or implant. The backing material may be incorporated in situ or adhered to the surgical adjunct or implant.

An example of the adherence during processing may include melt welding commonly used to bond thermoplastic materials. In this process, a combination of temperature, pressure, and time are controlled to have diffusion of the thermoplastics into each other. This process can be used to a lesser extent between a thermoplastic and thermoset material where the thermoplastic material flows around a geometric feature on the thermoset material and a mechanical bond is formed.

The stapling adjuncts or damping implants will likely be composed of either a thermoplastic or thermoset material. In the case of a thermoplastic material, there are limitations in retaining the desired shape with the thermal processing as the material may flow during processing. Conversely, the incorporation of a backing material with a thermoset is limited during thermal processing as the material will not flow and form an intimate bond interface.

Another limiting case for melt welding in the case of absorbable materials (e.g., a polyurethane foam) is the potential degradation during processing. Many absorbable materials, particularly faster absorbing materials, are thermally labile and thus may lose mechanical properties, generate degradant species, etc. during processing.

As such, there are limitations associated with (i) utilizing thermally labile materials, (ii) an inability to spatially modulate adhesive properties, and (iii) adherence limited to mechanical encapsulation for thermoset/thermoplastic combinations. Thus, there are inefficiencies for backing material incorporation a surgical adjunct or damping implant. Therefore, alternative methods and materials are discussed herein.

Additionally, films used to attach a surgical adjunct to a staple cartridge deck may be mechanically attached to the cartridge deck along with the surgical adjunct to prevent early release of the surgical adjunct from a staple cartridge deck.

Additionally, the surgical adjunct itself can be designed of made to include a gradient or double gradient of pore diameters in order to help maintain both compressive and tensile properties needed to create a hemostatic seal and also strong enough to allow a surgeon to grasp and manipulate the tails of the cushion. Such a morphology would mimic that of bone or other materials found in nature.

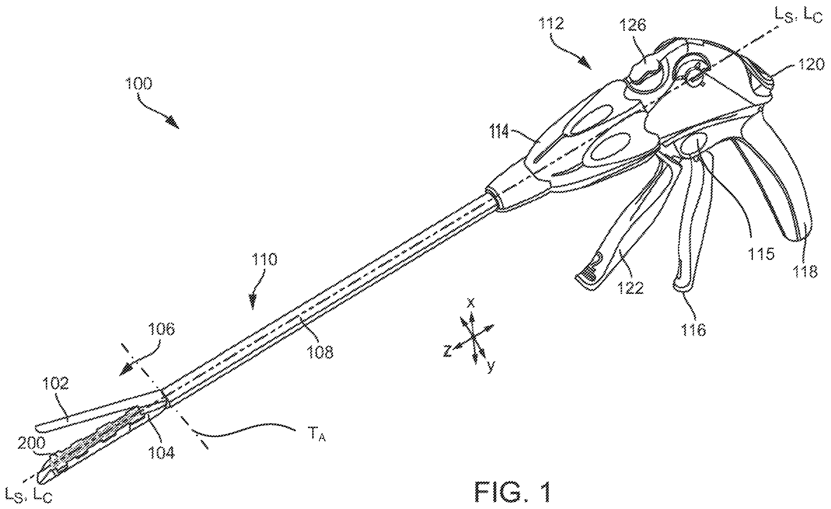

FIG. 1 illustrates an exemplary surgical stapling and severing device 100 suitable for use with an implantable adjunct. The illustrated surgical stapling and severing device 100 includes end effector 106 having an anvil 102 that is pivotably coupled to an elongate channel 104. As a result, the staple applying assembly 106 can move between an open position, as shown in FIG. 1, and a closed position in which the anvil 102 is positioned adjacent to the elongate channel 104 to engage tissue therebetween. The end effector 106 can be attached at its proximal end to an elongate shaft 108 forming an implement portion 110. When the end effector 106 is closed, or at least substantially closed, (e.g., the anvil 102 moves from the open position in FIG. 1 toward the elongate channel) the implement portion 110 can present a sufficiently small cross-section suitable for inserting the end effector 106 through a trocar. While the device 100 is configured to staple and sever tissue, surgical devices configured to staple but not sever tissue are also contemplated herein.

In various instances, the end effector 106 can be manipulated by a handle 112 connected to the elongate shaft 108. The handle 112 can include user controls such as a rotation knob 114 that rotates the elongate shaft 108 and the end effector 106 about a longitudinal axis (Ls) of the elongate shaft 108 and an articulation control 115 that can articulate the end effector 106 about an articulate axis (T_{A}) that is substantially transverse to the longitudinal axis (Ls) of the elongate shaft 108. Further controls include a closure trigger 116 which can pivot relative to a pistol grip 118 to close the end effector 106. A closure release button 120 can be outwardly presented on the handle 112 when the closure trigger 116 is clamped such that the closure release button 120 can be depressed to unclamp the closure trigger 116 and open the end effector 106, for example. Handle 112 may also take the form of an interface for connection to a surgical robot.

In some examples, a firing trigger 122, which can pivot relative to the closure trigger 116, can cause the end effector 106 to simultaneously sever and staple tissue clamped therein. The firing trigger 122 may be a powered, require force from a user to engage, or some combination thereof. A manual firing release lever 126 can allow the firing system to be retracted before full firing travel has been completed, if desired, and, in addition, the firing release lever 126 can allow a surgeon, or other clinician, to retract the firing system in the event that the firing system binds and/or fails.

Additional details on the surgical stapling and severing device 100 and other surgical stapling and severing devices suitable for use with the present disclosure are described, for example, in U.S. Pat. No. 9,332,984 and in U.S. Patent Publication No. 2009/0090763. Further, the surgical stapling and severing device need not include a handle, but instead can have a housing that is configured to couple to a surgical robot, for example, as described in U.S. Patent Publication No. 2019/0059889.

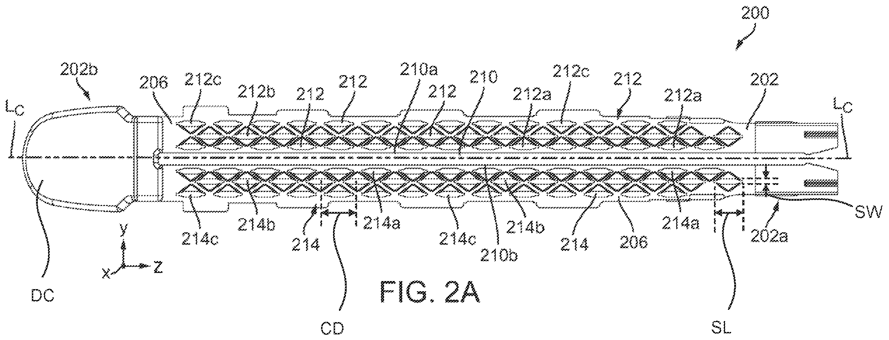

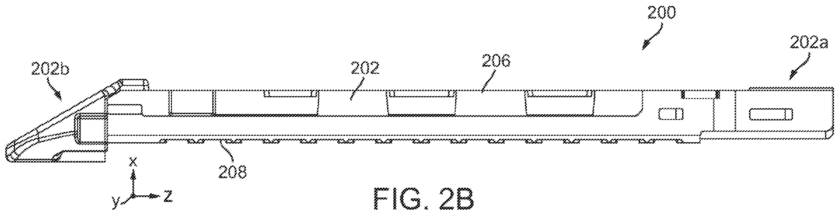

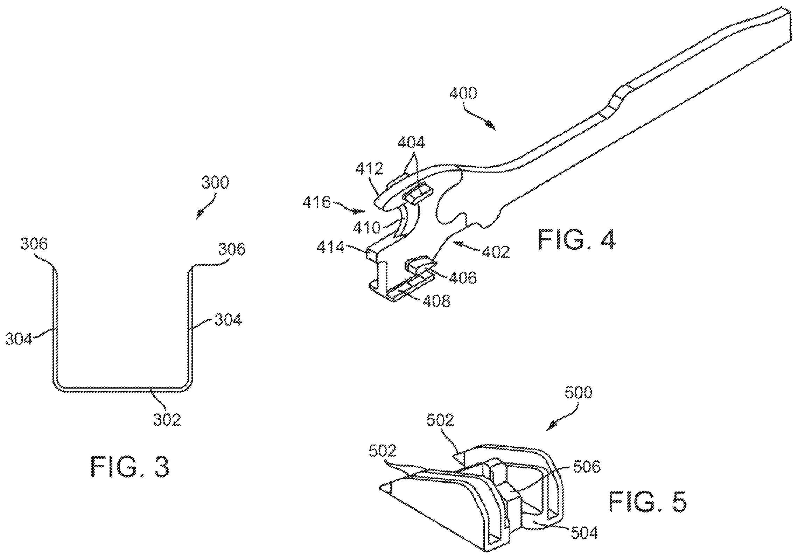

As further shown in FIG. 1, a staple cartridge 200 can be utilized with the instrument 100. In use, the staple cartridge 200 is placed within and coupled to the elongate channel 104. While the staple cartridge 200 can have a variety of configurations, in this illustrated example, the staple cartridge 200, which is shown in more detail in FIGS. 2A-2B, has a proximal end 202a and a distal end 202b with a cartridge longitudinal axis (LC) extending therebetween. As a result, when the staple cartridge 200 is inserted into the elongate channel 104 (FIG. 1), the longitudinal axis (LC) is substantially or approximately parallel with the longitudinal axis (LS) of the elongate shaft 108. Further, the staple cartridge 200 includes a longitudinal slot 210 defined by two opposing walls 210a, 210b and configured to receive at least a portion of a firing member of a firing assembly, like firing assembly 400 in FIG. 4, as discussed further below. As shown, the longitudinal slot 210 extends from the proximal end 202a toward the distal end 202b of the staple cartridge 200. It is also contemplated herein that in other examples, the longitudinal slot 210 can be omitted.

The illustrated staple cartridge 200 includes staple cavities 212, 214 defined therein, in which each staple cavity 212, 214 is configured to removably house at least a portion of a staple (not shown). The number, shape, and position of the staple cavities can vary and can depend at least on the size and shape (e.g., mouth-like shape) of the staples to be removably disposed therein. In this illustrated example, the staple cavities are arranged in two sets of three longitudinal rows, in which the first set of staple cavities 212 is positioned on a first side of the longitudinal slot 210 and the second set of staple cavities 214 is positioned on a second side of the longitudinal slot 210. On each side of the longitudinal slot 210, and thus for each set of rows, a first longitudinal row of staple cavities 212a, 214a extends alongside the longitudinal slot 210, a second row of staple cavities 212b, 214b extends alongside the first row of staple cavities 212a, 214a, and a third row of staple cavities 212c, 214c extends alongside the second row of staple cavities 212b, 214b. Each row may be approximately parallel and the staple cavities that make up the rows may be approximately parallel in orientation with the longitudinal slot 210. As shown in FIGS. 2A, each staple cavity 212, 214 may include a maximum length SL of about 3.099 mm (0.122 inches) to about 3.150 mm (0.124 inches) and a maximum width SW of about 0.584 mm (0.023 inches) to about 0.686 mm (0.027 inches). In addition, at least the centers of two adjacent cavities 212, 214 are spaced apart by about 4.013 mm (0.158 inches).

The staples releasably stored in the staple cavities 212, 214 can have a variety of configurations. An exemplary staple 300 that can be releasably stored in each of the staple cavities 212, 214 is illustrated in FIG. 3 in its unfired (pre-deployed, unformed) configuration. The illustrated staple 300 includes a crown (base) 302 and two legs 304 extending from each end of the crown 302. In this example, the crown 302 extends in a linear direction and the staple legs 304 have the same unformed height. Further, prior to the staples 300 being deployed, the staple crowns 302 can be supported by staple drivers that are positioned within the staple cartridge 200 and, concurrently, the staple legs 304 can be at least partially contained within the staple cavities 212, 214. Further, the staple legs 304 can extend beyond a top surface, like top surface 206, of the staple cartridge 200 when the staples 300 are in their unfired positions. In certain instances, as shown in FIG. 3, the tips 306 of the staple legs 304 can be pointed and sharp which can incise and penetrate tissue.

In use, staples 300 can be deformed from an unfired position into a fired position such that the staple legs 304 move through the staple cavities 212, 214, penetrate tissue positioned between the anvil 102 and the staple cartridge 200, and contact the anvil 102. As the staple legs 304 are deformed against the anvil 102, the legs 304 of each staple 300 can capture a portion of the tissue within each staple 300 and apply a compressive force to the tissue. Further, the legs 304 of each staple 300 can be deformed downwardly toward the crown 302 of the staple 300 to form a staple entrapment area in which the tissue can be captured therein. In various instances, the staple entrapment area can be defined between the inner surfaces of the deformed legs and the inner surface of the crown of the staple. The size of the entrapment area for a staple can depend on several factors such as the length of the legs, the diameter of the legs, the width of the crown, and/or the extent in which the legs are deformed, for example.

In some examples, all of the staples disposed within the staple cartridge 200 can have the same unfired (pre-deployed, unformed) configuration. In other examples, the staples can include at least two groups of staples each having a different unfired (pre-deployed, unformed) configuration, e.g., varying in height and/or shape, relative to one another, etc.

Referring back to FIGS. 2A-2B, the staple cartridge 200 extends from a top surface or deck surface 206 to a bottom surface 208, in which the top surface 206 is configured as a tissue-facing surface and the bottom surface 208 is configured as a channel-facing surface. As a result, when the staple cartridge 200 is inserted into the elongate channel 104, as shown in FIG. 1, the top surface 206 faces the anvil 102 and the bottom surface 208 (obstructed) faces the elongate channel 104.

With reference to FIGS. 4 and 5, a firing assembly such as, for example, firing assembly 400, can be utilized with a surgical stapling and severing device, like device 100 in FIG. 1. The firing assembly 400 can be configured to advance a wedge sled 500 having wedges 502 configured to deploy staples from the staple cartridge 200 into tissue captured between an anvil, like anvil 102 in FIG. 1, and a staple cartridge, like staple cartridge 200 in FIG. 1. Furthermore, an E-beam 402 at a distal portion of the firing assembly 400 may fire the staples from the staple cartridge. During firing, the E-beam 402 can also cause the anvil to pivot towards the staple cartridge, and thus move the end effector from the open position towards a closed position. The illustrated E-beam 402 includes a pair of top pins 404, a pair of middle pins 406, which may follow a portion 504 of the wedge sled 500, and a bottom pin or foot 408. The E-beam 402 can also include a sharp cutting edge 410 configured to sever the captured tissue as the firing assembly 400 is advanced distally, and thus towards the distal end of the staple cartridge. In addition, integrally formed and proximally projecting top guide 412 and middle guide 414 bracketing each vertical end of the cutting edge 410 may further define a tissue staging area 416 assisting in guiding tissue to the sharp cutting edge 410 prior to being severed. The middle guide 414 may also serve to engage and fire the staples within the staple cartridge by abutting a stepped central member 506 of the wedge sled 500 that effects staple formation by the end effector 106.

In use, the anvil 102 in FIG. 1 can be moved into a closed position by depressing the closure trigger in FIG. 1 to advance the E-beam 402 in FIG. 4. The anvil 102 can position tissue against at least the top surface 206 of the staple cartridge 200 in FIGS. 2A-2B. Once the anvil has been suitably positioned, the staples 300 in FIG. 3 disposed within the staple cartridge can be deployed.

To deploy staples from the staple cartridge, as discussed above, the sled 500 in FIG. 5 can be moved from the proximal end toward a distal end of the cartridge body, and thus, of the staple cartridge. As the firing assembly 400 in FIG. 4 is advanced, the sled can contact and lift staple drivers within the staple cartridge upwardly within the staple cavities 212, 214. In at least one example, the sled and the staple drivers can each include one or more ramps, or inclined surfaces, which can co-operate to move the staple drivers upwardly from their unfired positions. As the staple drivers are lifted upwardly within their respective staple cavities, the staples are advanced upwardly such that the staples emerge from their staple cavities and penetrate into tissue. In various instances, the sled can move several staples upwardly at the same time as part of a firing sequence.

As indicated above, the stapling device can be used in combination with a compressible adjunct. A person skilled in the art will appreciate that, while adjuncts are shown and described below, the adjuncts disclosed herein can be used with other surgical instruments and need not be coupled to a staple cartridge as described. Further, a person skilled in the art will also appreciate that the staple cartridges need not be replaceable.

As discussed above, with some surgical staplers, a surgeon is often required to select the appropriate staples having the appropriate staple height for tissue to be stapled. For example, a surgeon will utilize tall staples for use with thick tissue and short staples for use with thin tissue. In some instances, however, the tissue being stapled does not have a consistent thickness and thus, the staples cannot achieve the desired fired configuration for every section of the stapled tissue (e.g., thick and thin tissue sections). The inconsistent thickness of tissue can lead to undesirable leakage and/or tearing of tissue at the staple site when staples with the same or substantially greater height are used, particularly when the staple site is exposed to intra-pressures at the staple site and/or along the staple line.

Accordingly, various examples of adjuncts are provided that can be configured to compensate for varying thickness of tissue that is captured within fired (deployed) staples to avoid the need to take into account staple height when stapling tissue during surgery. That is, the adjuncts described herein can allow a set of staples with the same or similar heights to be used in stapling tissue of varying thickness (e.g., from thin to thick tissue) while also, in combination with the adjunct, providing adequate tissue compression within and between fired staples. Thus, the adjuncts described herein can maintain suitable compression against thin or thick tissue stapled thereto to thereby minimize leakage and/or tearing of tissue at the staple sites. In addition, exemplary adjuncts described herein may be configured to be essentially fully absorbed in the body over a period of 100 to 300 days depending on implanted location and tissue health.

Alternatively, or in addition, the adjuncts can be configured to promote tissue ingrowth. In various instances, it is desirable to promote the ingrowth of tissue into an implantable adjunct, to promote the healing of the treated tissue (e.g., stapled and/or incised tissue), and/or to accelerate the patient's recovery. More specifically, the ingrowth of tissue into an implantable adjunct may reduce the incidence, extent, and/or duration of inflammation at the surgical site. Tissue ingrowth into and/or around the implantable adjunct may, for example, manage the spread of infections at the surgical site. The ingrowth of blood vessels, especially white blood cells, for example, into and/or around the implantable adjunct may fight infections in and/or around the implantable adjunct and the adjacent tissue. Tissue ingrowth may also encourage the acceptance of foreign matter (e.g., the implantable adjunct and the staples) by the patient's body and may reduce the likelihood of the patient's body rejecting the foreign matter. Rejection of foreign matter may cause infection and/or inflammation at the surgical site.

In general, the adjuncts provided herein are designed and positioned atop a staple cartridge, like staple cartridge 200. When the staples are fired (deployed) from the cartridge, the staples penetrate through the adjunct and into tissue. As the legs of the staple are deformed against the anvil that is positioned opposite the staple cartridge, the deformed legs capture a portion of the adjunct and a portion of the tissue within each staple. That is, when the staples are fired into tissue, at least a portion of the adjunct becomes positioned between the tissue and the fired staple. While the adjuncts described herein can be configured to be attached to a staple cartridge, it is also contemplated herein that the adjuncts can be configured to mate with other instrument components, such as an anvil of a surgical stapler. A person of ordinary skill will appreciate that the adjuncts provided herein can be used with replaceable cartridges or staple reloads that are not cartridge based.

### Methods of Stapling Tissue

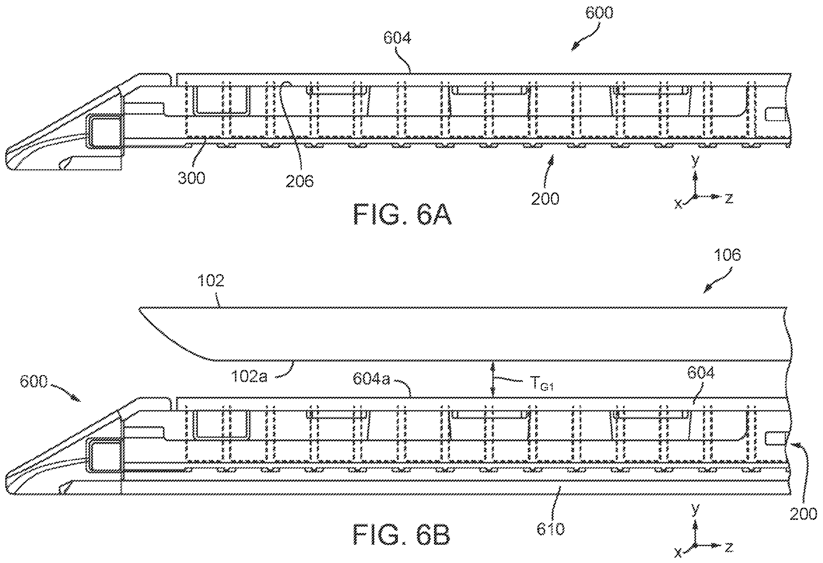

FIGS. 6A-6B illustrate an exemplary example of a stapling assembly 600 that includes a staple cartridge 200 and an adjunct 604. For sake of simplicity, the adjunct 604 is generally illustrated in FIGS. 6A-6B, and various configurations of the adjunct are described in more detail below. As shown, the adjunct 604 is positioned against the staple cartridge 200. While partially obstructed in FIGS. 6A-6B, the staple cartridge 200 includes staples 300, that are configured to be deployed into tissue. The staples 300 can have any suitable unformed (pre-deployed) height.

In the illustrated example, the adjunct 604 can be mated to at least a portion of the top surface or deck surface 206 of the staple cartridge 602. In some examples, the top surface 206 of the staple cartridge 200 can include one or more surface features which can be configured to engage the adjunct 604 to avoid undesirable movements of the adjunct 604 relative to the staple cartridge 200 and/or to prevent premature release of the adjunct 604 from the staple cartridge 200. Exemplary surface features are described further below and in U.S. Patent Publication No. 2016/0106427.

FIG. 6B shows the stapling assembly 600 placed within and coupled to the elongate channel 610 of surgical end effector 106. The anvil 102 is pivotally coupled to the elongate channel 610 and is thus moveable between open and closed positions relative to the elongate channel 610, and thus the staple cartridge 200. The anvil 102 is shown in a closed position in FIG. 6B and illustrates a tissue gap T_{G1} created between the staple cartridge 602 and the anvil 612. More specifically, the tissue gap T_{G1} is defined by the distance between the tissue-compression surface 102a of the anvil 102 (e.g., the tissue-engaging surface between staple forming pockets in the anvil) and the tissue-contacting surface 604a of the adjunct 604. In this illustrated example, both the tissue-compression surface 102a of the anvil 102 and the tissue-contacting surface 604a of the adjunct 604 is planar, or substantially planar (e.g., planar within manufacturing tolerances). As a result, when the anvil 102 is in a closed position, as shown in FIG. 6B, the tissue gap T_{G1} is generally uniform (e.g., nominally identical within manufacturing tolerances) when no tissue is disposed therein. In other words, the tissue gap T_{G1} is generally constant (e.g., constant within manufacturing tolerances) across the end effector 106 (e.g., in the y-direction). In other examples, the tissue-compression surface of the anvil can include a stepped surface having longitudinal steps between adjacent longitudinal portions, and thus create a stepped profile (e.g., in the y-direction). In such examples, the tissue gap T_{G1} can be varied.

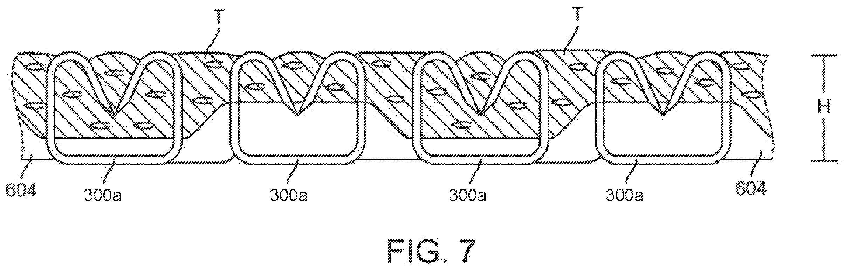

The adjunct 604 is compressible to permit the adjunct to compress to varying heights to thereby compensate for different tissue thickness that are captured within a deployed staple. The adjunct 604 has an uncompressed (undeformed), or pre-deployed, height and is configured to deform to one of a plurality of compressed (deformed), or deployed, heights. For example, the adjunct 604 can have an uncompressed height which is greater than the fired height of the staples 300 disposed within the staple cartridge 200 (e.g., the height (H) of the fired staple 300a in FIG. 7). That is, the adjunct 604 can have an undeformed state in which a maximum height of the adjunct 604 is greater than a maximum height of a fired staple (e.g., a staple that is in a formed configuration).

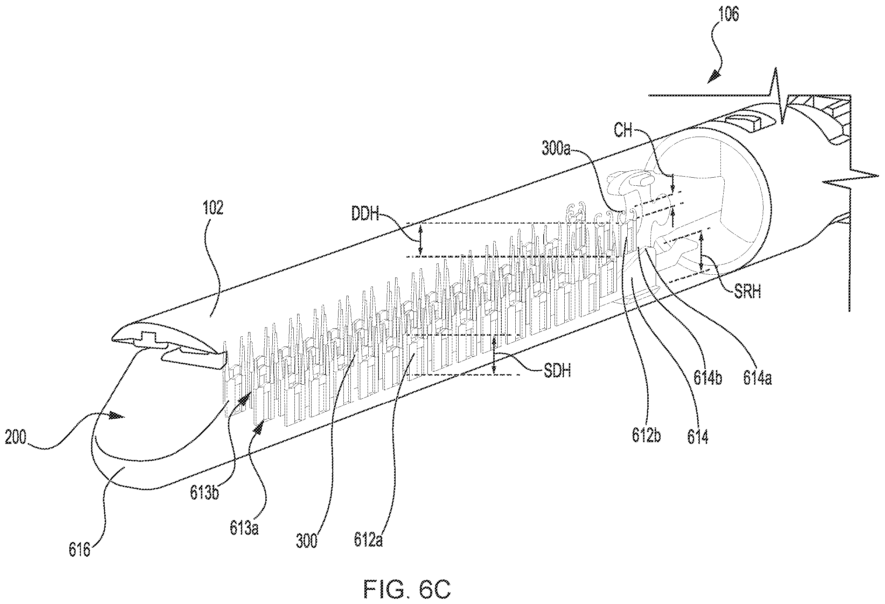

As shown in FIG. 6C, the staple cartridge 200 includes a sled 614 and a plurality of drivers 612 configured to drive one or more staples in an upward direction to deploy the staples when a user presses a firing trigger 122 shown in FIG. 1. Once the firing trigger 122 is pressed, the sled 614 moves toward the distal end 616 of the end effector 106 contacting one or more drivers 612a, 612b at a time forcing the one or more drivers 612a, 612b upward along with one or more corresponding staples 300 upward to form fired staple 300a and to capture a material, such as tissue (T), (see FIG. 7.) between the anvil 104 and the fired staple 300a. The cartridge 200 may include a first row 613a of single drivers 612a that corresponds to driving staples 300 positioned in the third row of staple cavities 212c, 214c and a second row 613b of double drivers 612b that corresponds to driving staples 300 positioned in a first row of staple cavities 212a, 214b and a third row 212a, 212b (see FIG. 2A for staple cavities).

Referring to FIG. 6C, one or more single drivers 612a may have a height SDH of about 1.118 mm (0.044 inches) to about 1.880 mm (0.074 inches), such as about 1.270 mm (0.050 inches) to about 1.727 mm (0.068 inches), about 1.372 mm (0.054 inches), or about 1.52 mm (0.06 inches). One or more double drivers 612b may have a height DDH of about 1.118 mm (0.044 inches) to about 1.880 mm (0.074 inches), such as about 1.270 mm (0.050 inches) to about 1.727 mm (0.068 inches), about 1.372 mm (0.054 inches), or about 1.52 mm (0.06 inches). The sled 614 may have a least a first rail 614a corresponding to the single drivers 612a positioned in the first row 613a and a second rail 614b corresponding to the double drivers 612b positioned in the second row 613b. The first rail 614a may have a rail height SRH of about 4.166 mm (0.164 inches) or about 4.242 mm (0.167 inches) and engages with the single driver 612a. The second rail 614b may have a rail height DRH of about 3.556 mm (0.140 inches) to about 4.115 mm (0.162 inches) such as about 3.785 mm (0.149 inches) or about 3.861 mm (0.152 inches) and engages with the double driver 612b. Once the staples 300 are deployed, they form a fired staple 300a with a crush height CH of about 2.03 mm (0.08 inches) to about 3.05 mm (0.12 inches) such as about 2.5 mm (0.1 inches).

As shown in FIG. 7, when the staples 300 are fired, tissue (T) and a portion of the adjunct 604 are captured by the fired (formed) staples 300a. The fired staples 300a each define the entrapment area therein, as discussed above, for accommodating the captured adjunct 604 and tissue (T). The entrapment area defined by a fired staple 300a is limited, at least in part, by a height (H) of the fired staple 300a.

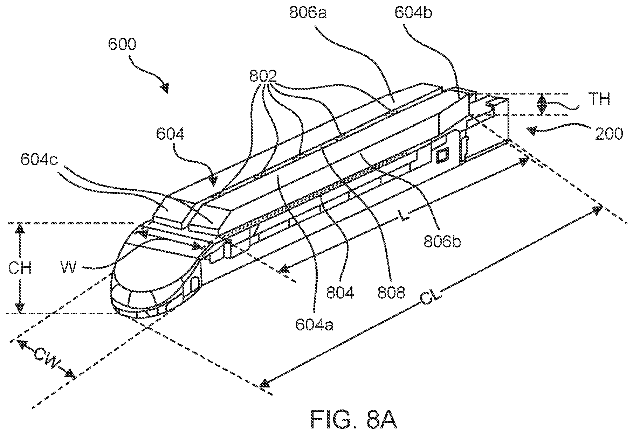

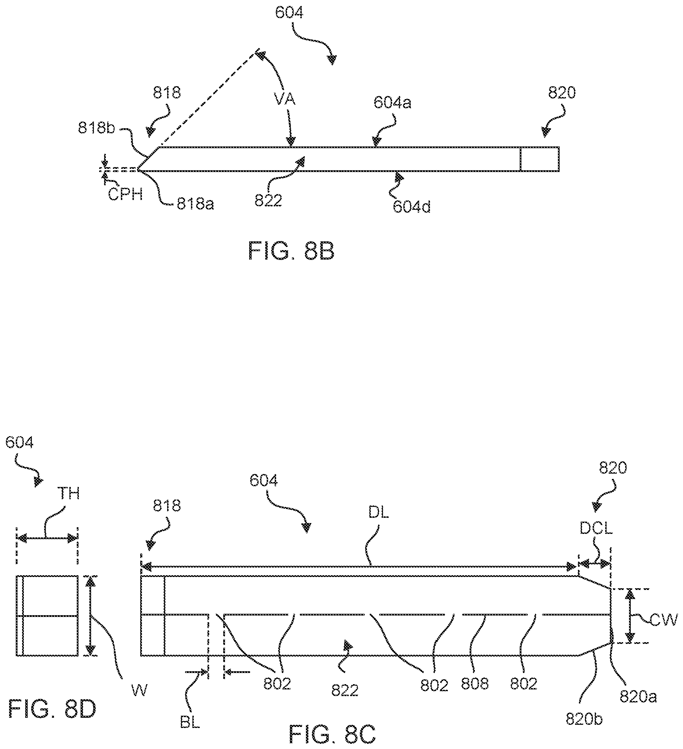

FIG. 8A illustrates a perspective view of a staple cartridge assembly 600 with an adjunct 604 and a staple cartridge 200. The adjunct 604 has a tissue contacting surface 604a, a proximal end 604a, and a distal end 604b. The adjunct 604 may include a slot/slit 808 separating or partially separating two parallel portions of the adjunct 604. In one example, adjunct 604 may include a slot 808 separating two parallel portions of the adjunct 604, while in another example, adjunct 604 may include a slit 808 separating two parallel portions of the adjunct 604 and also one or more bridges (e.g., five bridges) 802 connecting the two parallel portions of the adjunct 604. At least one bridge has a length in the longitudinal direction of about 0.889 mm (0.035 inches) to about 1.168 mm (0.046 inches). The adjunct 604 has a length L of about 40 mm to about 80 mm, such as about 60 mm to about 65 mm, about 66.04 mm to about 66.3 mm, about 45 mm to about 55 mm, or about 51.12 mm to about 51.38 mm. The adjunct 604 has a width W of about 8 mm to about 12 mm, such as about 9.75 mm to about 10.25 mm or about 10.025 mm to about 10.035 mm. The adjunct 604 may also have a thickness or height TH of about 2.5 mm to about 3.5 mm, such as about 2.85 mm to about 3.15 mm or about 2.95 mm to about 3.05 mm.

The cartridge 200 has a height CH of about 6.3 mm to about 8.1 mm, a width CW of about 8.9 mm to about 14 mm, and a length CL of about 80 to about 90mm such as about 86.7 mm.

Referring to FIGS. 8B-8D, the adjunct 604 has a lower surface 604d and may have a distal chamfered portion 818, a proximal chamfered portion 820, and a center portion 822. The distal chamfered portion 818 has a vertical portion 818a having a height CPH of about 0.229 mm (0.009 inches) to about 0.737 mm (0.029 inches), such as about 0.483 mm (0.019 inches). The distal chamfered portion 818 may have an angled portion 818b proximal the vertical portion 818a. The angled portion 818b has a slope VA of about 30 degrees to about 60 degrees, such as about 45 degrees, measured from the tissue contacting surface 604a.

Referring to FIG. 8C, the distal chamfered portion 818 and the center portion 822 has a combined length DL of about 57.15 mm (2.25 inches) to about 62.23 mm (2.45 inches), such as about 59.69 mm (2.35 inches). The proximal chamfered portion 820 has an angled portion 820b with a length DCL of about 2.5 mm (0.1 inches) to about 7.6 mm (0.3 inches). In addition, the proximal chamfered portion 820 has a horizontal portion 820a and an angled portion 820n. The horizontal portion 820a may have a width CW of about 6.86 mm (0.27 inches) to about 7.37 mm (0.29 inches), such as about 7.11 mm (0.28 inches).

In some examples, the adjunct 604 includes one or more slits 808 with two or more bridges 802 spaced apart by a bridge length BL of about 0.889 mm (0.035 inches) to about 1.143 mm (0.045 inches) such as about 1.02 mm (0.04 inches).

Referring back to FIG. 8A, the staple cartridge 200 may include one or more raised ledges 804 along one or more sides of the adjunct 602 to help align the adjunct 604 on the deck of the staple cartridge 200.

The surgical adjunct 604 may have one or more of the properties described below to enable the adjunct to be flexible when in vivo, but yet remain in a certain position attached to the cartridge when outside of the body. For example, the polyurethane may server to create an adjunct 604 that is flexible when going into the body but "sets" to its final mechanical properties as the plasticizer is absorbed in vivo. In some examples, a plasticizer may be added to the polyurethane foam to lower its glass transition temperature to be within the below described ranges as well as conform to the other listed properties. Regardless, an adjunct 604 having one or more of the below properties consistently creates a hemostatic or near hemostatic seal on tissue.

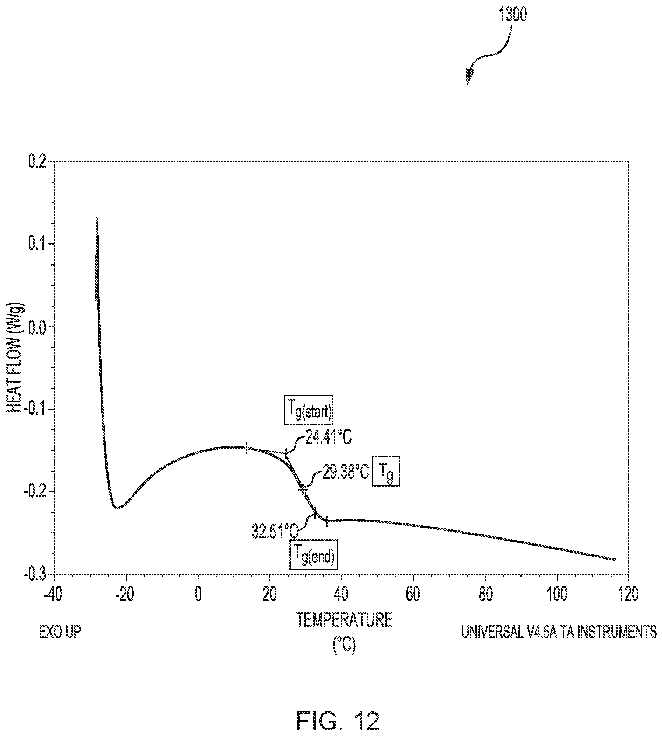

The surgical adjunct 604 may include a polyurethane foam with or without a plasticizer where the glass transition temperature of the surgical adjunct 604 is about 0°C to about 40°C (e.g., about 19.4°C), such as about 7.5°C to about 22.5°C or about 12.5°C to about 17.5°C. The glass transition temperature of the adjunct 604 is obtained by using a standard differential scanning calorimetry (DSC) system. Using the DSC system with its output shown in FIG. 12, an adjunct 604 was equilibrated at about -40°C, heated at about 40°C/min to about 120°C, held isothermally for about 1 minute, cooled at about 40°C/min to about -40°C, held isothermally for about 1 minute, and then heated at about 10°C/min to about 120°C, where the glass transition temperature T_{g} was measured and recorded by the DSC system.

The surgical adjunct 604 may include a volumetric ratio of the polyurethane foam to the total volume of the adjunct 604 of about 0.125 to about 0.325, such as about 0.175 to about 0.225 or about 0.19 to about 0.21. The total volume may include air (from pores of the foam) or other material beside the foam structures.

The plasticizer may include one or more of a low molecular weight glycol, polyethylene glycol, polyvinylpyrrolidone, dibutyl sebacate, glyceryl triacetate, glyceryl behenate, hexanoic acid, decanoic acid, octadecanoic acid, boric ester, and a fatty acid. In some examples, the plasticizer includes one or more fatty acids.

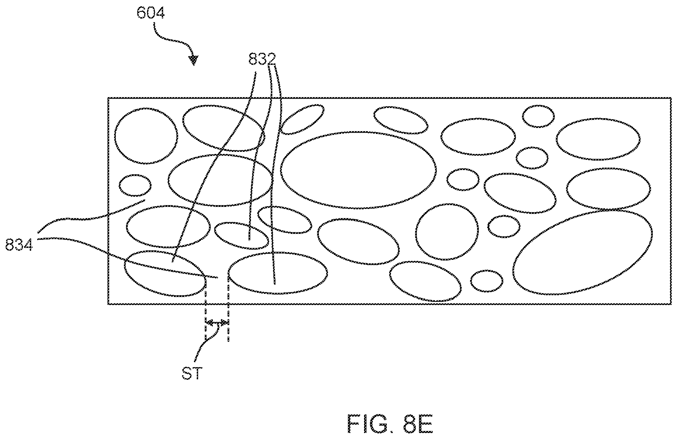

Referring to FIG. 8E, the adjunct 604 may have pores 832 with a median pore size of about 0.025 mm³ to about 0.300 mm³, such as about 0.022 mm³. In some examples, the adjunct 604 may have one or more struts 834 between the pore 832 that provide support and strength to the adjunct 604. In particular, the adjunct 604 may include a plurality of struts 834, having a median strut thickness ST of about 0.025 mm to about 0.300 mm, such as about 0.08 mm.

In some examples, the adjunct 604 includes a polydioxanone (PDO) film disposed on one or more surfaces of the polyurethane foam. In some examples, the PDO film is adhered to at least a bottom or crown side of the adjunct 604. In some examples, the PDO film has a thickness of about 20 µm to about 100 µm, such as about 40 µm.

The adjunct 604 may have a compression strength of about 30 kPa to about 70 kPa, such as about 30 kPa to about 60 kPa (e.g., about 42 kPa), about 30 kPa to about 50 kPa, about 32.5 kPa to about 37.5 kPa. In order to test compression strengths, an adjunct 604 was placed in a humid warm environment at approximately 37°C, compressed to a first height, then a second height shorter than the first height, and then released back to the first height at which point the adjunct's compression strength was measured.

In some examples, the adjunct 604 may have a tensile strength of about 30 kPa to about 90 kPa such as about 45 kPa to about 85 kPa or about 55 kPa to about 75 kPa. In some examples, the adjunct 604 will have tensile strength of about 110 kPa to about 150 kPa.

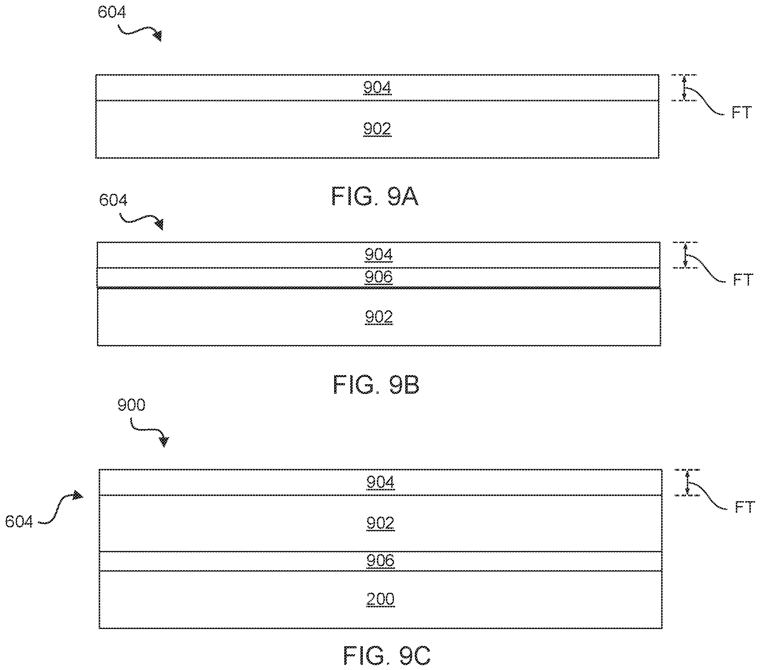

Referring to FIG. 9A, the surgical adjunct 604 may include a bioabsorbable material 902 (e.g., bioabsorbable foam such a polyurethane foam) and a film 904 having a film thickness FT disposed on the absorbable material. This film thickness FT of the film 904 may range from about 0.0076 mm (0.0003 inches) to about 0.254 mm (0.010 inches). For example, the film may be a skin made of the same material as the bioabsorbable materials 902 and may include a film thickness FT with a range of about 0.025 mm (0.001 inches) to about 0.254 mm (0.010 inches). In other instances, the film 904 can be a mostly continuous surface on the staple crown side of the bioabsorbable materials 902 to help guide the staples 300 when firing and may have a film thickness ranges from about 0.00991 mm (0.00039) inches to about 0.0991 mm (0.0039 inches) (e.g., about 0.01168 mm (0.00046 inches) to about 0.508 mm (0.020 inches)). This film 904 may include pores with diameters in the range of about of about 0.0013 mm (0.0005 inches) to about 0.013 mm (0.005 inches) when formed from the same material (e.g., polyurethane or polyurethane foam) as the bioabsorbable material 902. In some examples, when the film 904 comprises the same bioabsorbable material (e.g., polyurethane) the film 904 may have a higher density than then bioabsorbable material 902. When the film 904 includes other materials, the film thickness FT may range from about 0.025 mm (0.001 inches) to about 0.076 mm (0.003 inches). For example, the film 904 may include polydioxanone (PDO), poly(delta-gluconolactone) (PGL-1), poly(glycolide/l-lactide) (PGL-2), polyglycolic acid (PGA), a glycolide and epsilon caprolactone copolymer (PGCL), a glycolide and l-lactide copolymer, urethane, polycaprolactone (PCL), polyglactin 370 (PG-370), polyglactin 185 (PG-185), or combinations thereof.

Referring to FIG. 9B, the surgical adjunct 604 may include a bioabsorbable material 902, and a film 904 disposed on the bioabsorbable material 902 with an adhesive 906 disposed between the film 940 and the bioabsorbable material 902. Referring to FIG. 9C, the surgical adjunct 604 may include a bioabsorbable material 902 disposed on a staple cartridge 200 with an adhesive 906 disposed between the bioabsorbable material 902 and the cartridge 200. Additionally, a film 904 is disposed on a surface of the bioabsorbable material 902. In some examples, the adhesive may include polyvinylpyrrolidone. In some examples, the adhesive 906 is bioabsorbable.

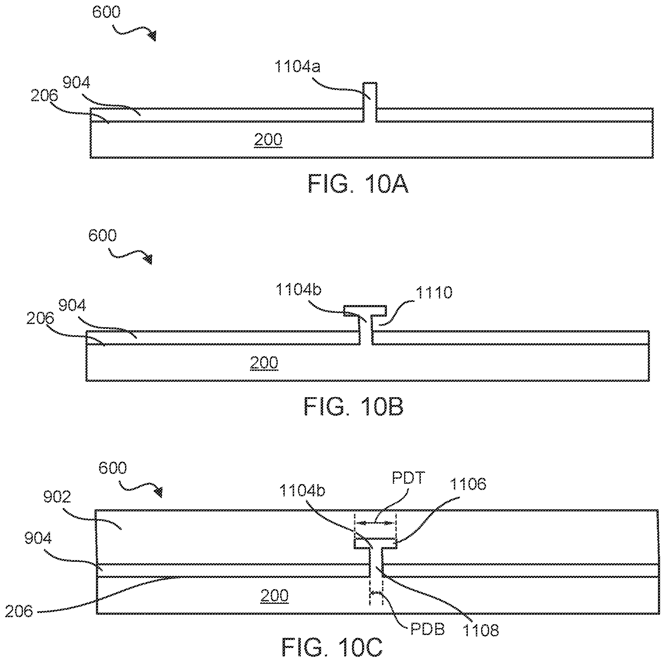

Referring to FIG. 10A, stapling assembly 600 may include a cartridge 200 that includes a deck 206 and at least one post 1104a extending away from that deck. The stapling assembly includes a film 904 disposed on deck 206. The at least one post 1104a may be heated to form a post 1104b with a top 1106 and a bottom 1108 as shown in FIG. 10B. As shown in FIG. 10C, the bioabsorbable material 902 may be disposed on the film 904 and within a gap 1110 between the top 1006 of the at least one post 1104b and the film 904. Since the top 1106 may have a diameter PDT that is greater than a diameter PDB of the bottom 1108, the bioabsorbable material 902 may be mechanically locked in place or attached to the stapling assembly 600.

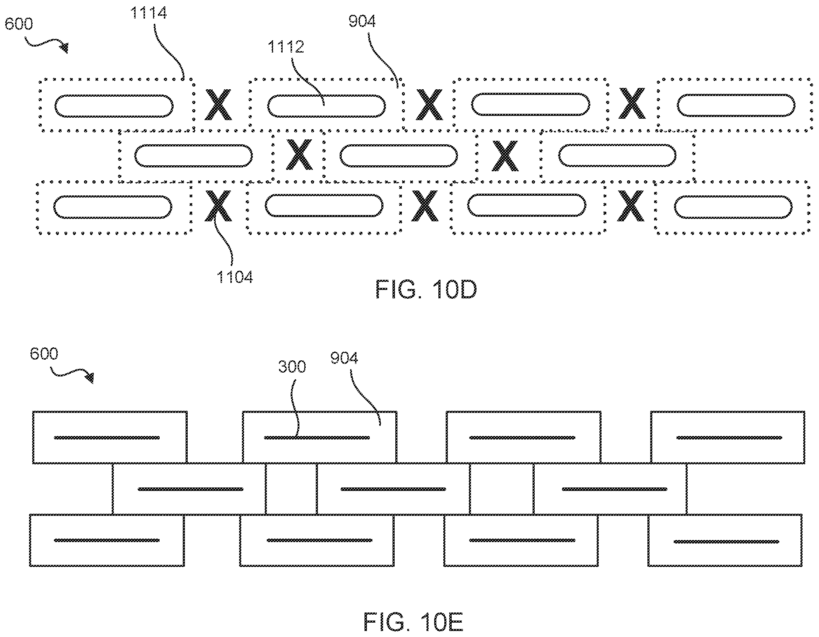

FIG. 10D shows a top view of an exemplary stabling assembly 600. As shown, the posts 1104 may be disposed between staple slots 1112 of the cartridge 200. In addition, the film 904 may include perforations 1114 at least partially surrounding or covering the staple slots 1112 and the posts 1104 to allow the film to support the staples 300 shown in FIG. 10E.

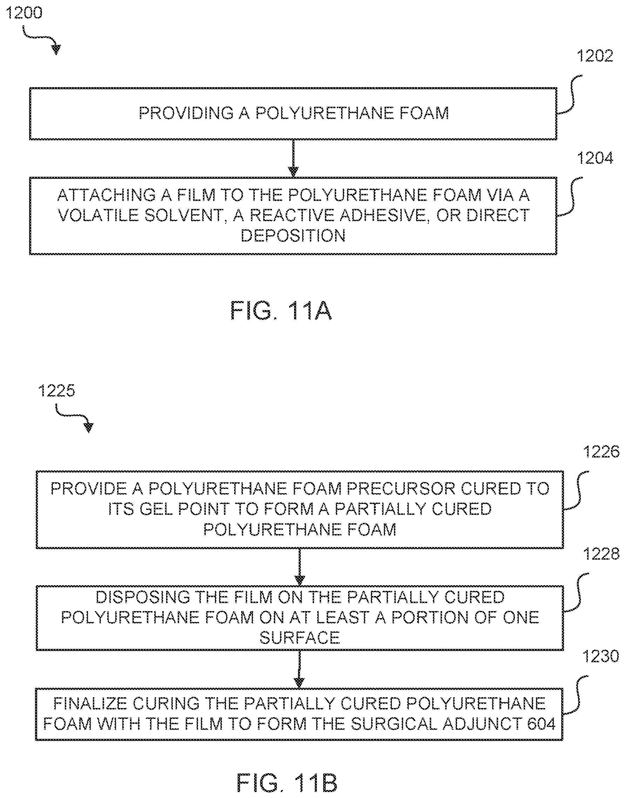

Referring to FIG. 11A, method 1200 relates to adding a film to a bioabsorbable material such as a polyurethane foam. The method includes providing 1202 a polyurethane foam and attaching 1204 a film to the polyurethane foam via a volatile solvent (e.g., solvent welding), a reactive adhesive, or direct deposition.

Solvent welding may be used to add a film to bioabsorbable material. For example, solvents such as ethyl acetate, dichloromethane, acetone may be used. Using this process eliminated the need for thermal processing and eliminated the need for spatial arrangement of bonding parts with the film.

Reactive adhesives may be used to add a film to the bioabsorbable material. Some reactive adhesives may include polyurethanes, epoxies, acrylates, etc. The reactive adhesive may be directly deposited onto the adjunct and/or the film. This can be patterned with both surface area and mass modulate the adhesive strength along and across the bioabsorbable material. Such an application may be useful in the case where selective release of the film is designed to minimize the overall bioabsorbable material (e.g., film only were contacted with staples with the remaining staying with the endoscopic instrument. In addition to the application process, the chemistry may be used to tune the adhesive properties. The reactive adhesive may be applied using inkjet printing, direct deposition, thermal spraying, cold dynamic spraying, cold spraying, electro spraying, ultrasonic spray coating, dip coating, screen printing, and spin coating. Using adhesives is beneficial because it is possible to bond two thermoset materials (film and bioabsorbable material) where material flow is not required. Using reactive adhesives may eliminate the need for thermal processing, eliminate need to arrange bonding points between film and bioabsorbable material, and allows for the modulating of adhesive forces across the surgical adjunct to create a selective release feature if needed.

Direct deposition may be used to directly apply a film to the bioabsorbable material. The film itself may be distributed in a solution or be composed of a reactive mixture that is subsequently applied to the bioabsorbable material. Direct depositions method for adding the film to the bioabsorbable material may include stereolithography, lithography, holographic printing, inject printing, direction deposition, thermal spraying, cold dynamic spraying, cold spraying, electro spraying, ultrasonic spray coating, dip coating, screen printing, and spin coating. Reactive mixtures for these processes, may include polyurethane, epoxy. Photocurable formulations may be used as well. These can include a photoinitiator, a solvent, inhibitors, photocurable oligomer or monomer, light absorber, and mixtures thereof. The advantages of directly applying the film include the ability to conformally apply the film, spatial control of the film, being able to easily apply the film, and flexibility in applying the film in terms of rate, thickness, etc. for different uses.

Referring to FIG. 11B, method 1225 relates to adding or forming a film when forming the polyurethane foam or in-situ. The method 1225 includes providing 1226 a polyurethane foam precursor cured to its gel point to form a partially cured polyurethane foam 902, disposing or forming the film 904 on the partially cured polyurethane foam on at least a portion of the one surface, finalize curing 1230 the partially cured polyurethane foam with the film to form the surgical adjunct 604.

When using thermoset materials (e.g., polyurethane foams) for surgical adjuncts, there is a potential to incorporate the material during the adjunct fabrication process. Before the completion of curing and/or specific stochiometric imbalance, the film may be applied to the adjunct. For best results, the addition of the film should be completed after the gel point of the thermoset material to ensure that the film does not constrain the adjunct formation. After applying the film, the curing process should resume to allow for chemical bonds between the thermoset material and the film. This will especially be the case where free hydroxyl groups are present in the case of polyurethane-based adjuncts. The chemical bonds would provide strength profiles that may exceed any adhesive interaction between the film and the thermoset material. Advantages to using this method include, elimination of thermal processing, potential covalent attachment between the film and the thermoset material, and the elimination of the need for a separate adhesive.

Additionally, the film such as PDO may be overmolded on the bioabsorbable material.

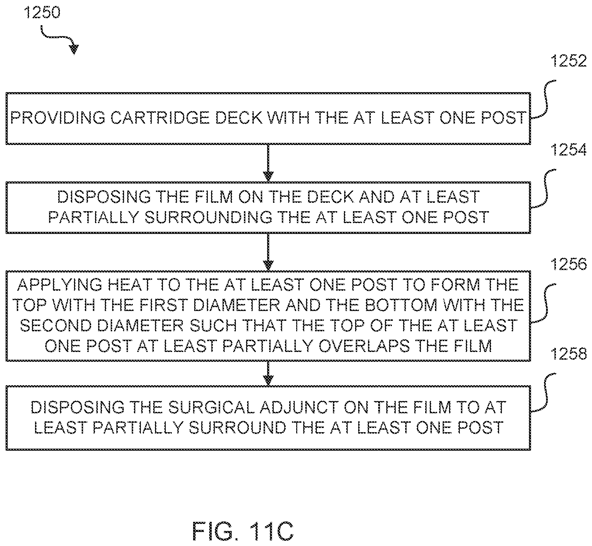

Referring to FIG. 11C, method 1250 of attaching a film 904 and bioabsorbable material 902 to a cartridge deck is illustrated. Method 1250 may include providing 1252 a cartridge deck 206 with the at least one post 1104, disposing 1254 the film on the deck and at least partially surrounding the at least one post 1104, applying 1256 heat to the at least one post 1104to form the top 1106 with the first diameter and the bottom 1108 with the second diameter such that the top 1106 of the at least one post 1104 at least partially overlaps the film 904. Method 1250 also includes disposing 1258 the surgical adjunct on the film 904 to at least partially surrounds the at least one post 1104.

## Claims

1. A stapling assembly (600), comprising:
a deck (206); and
a surgical adjunct (604) disposed on deck (206) and comprising a polyurethane foam (902) with a volumetric ratio of the polyurethane foam (902) to the total volume of the surgical adjunct (604) in a range of about 0.125 to about 0.325, wherein the surgical adjunct (604) comprises a film (904) disposed on at least one surface of the polyurethane foam (902), and wherein the film (904) has a thickness FT of about 0.0076 mm (0.0003 inches) to about 0.254 mm (0.010 inches) and has pore diameters of about 0.0127 mm (0.0005 inches) to about 0.127 mm (0.005 inches).

2. The stapling assembly of claim 1, wherein the film (904) comprises polyurethane (902) having a higher density than the polyurethane foam (902).

3. The stapling assembly of claim 1, wherein the film (904) is laminated on the at least one surface of the polyurethane foam and comprises an absorbable material selected from the group consisting of polydioxanone (PDO), poly(delta-gluconolactone) (PGL-1), poly(glycolide/l-lactide) (PGL-2), polyglycolic acid (PGA), a glycolide and epsilon caprolactone copolymer (PGCL), a glycolide and l-lactide copolymer, urethane, polycaprolactone (PCL), polyglactin 370 (PG-370), polyglactin 185 (PG-185), or combinations thereof.

4. The stapling assembly of claim 3, wherein the film (904) has a thickness FT of about 0.0076 mm (0.0003 inches) to about 0.076 mm (0.003 inches).

5. The stapling assembly of claim 3 or 4, wherein the film (904) is attached to the at least one surface of the polyurethane foam with an adhesive.

6. The stapling assembly of claim 3, wherein the absorbable material comprises polyglactin 370.

7. The stapling assembly of claim 1, wherein:
the deck (206) comprises a surface and at least one post (1104) extending away from the surface, the at least one post (1104) having a top (1106) with a first diameter PDT and a bottom (1108) with a second diameter PDB that is less than the first diameter PDT,
the film (904) is disposed on the surface and at least partially surrounds the at least one post (1104) with a gap (1110) between the top (1106) of the at least one post (1104) and the film (904); and
the surgical adjunct (604) is disposed on the film (904) and at least partially surrounding the at least one post.

8. The stapling assembly of claim 7, wherein the film (904) comprises a perforated pattern surrounding one or more staple slots (1112).

9. A method for making a surgical tool (200), comprising:
providing a surgical adjunct (604), comprising polyurethane foam (902) comprising a volumetric ratio of the polyurethane foam (902) to the total volume of the surgical adjunct (604) is in a range of about 0.125 to about 0.325; and
disposing or forming a film (904) on the polyurethane foam (902), wherein the film (904) has a thickness FT of about 0.0076 mm (0.0003 inches) to about 0.254 mm (0.010 inches) and has pore diameters of about 0.0127 mm (0.0005 inches) to about 0.127 mm (0.005 inches).

10. The method of claim 9, comprising:
disposing at least one volatile solvent (906) on at least a portion of at least one surface of the polyurethane foam (902);
disposing the film (904) on the at least one volatile solvent (906); and
dissolving a least a portion of the polyurethane foam (902) and the film (904) with the at least one volatile solvent to join the polyurethane foam (902) and the film (904).

11. The method of claim 9, comprising:
providing the polyurethane foam (902);
disposing a reactive adhesive (906) on at least a portion of the film (904), at least a portion of the polyurethane foam (902), or both; and
disposing the film on the at least polyurethane foam (902) such that the reactive adhesive (906) is sandwiched between the polyurethane foam (902) and the film (904).

## Patentansprüche

1. Klammeranordnung (600), umfassend:
ein Deck (206); und
ein chirurgisches Hilfsmittel (604), das auf dem Deck (206) angeordnet ist und umfassend einen Polyurethanschaum (902) mit einem Volumenverhältnis des Polyurethanschaums (902) zu dem Gesamtvolumen des chirurgischen Hilfsmittels (604) in einem Bereich von etwa 0,125 bis etwa 0,325, wobei das chirurgische Hilfsmittel (604) einen Film (904) umfasst, der auf mindestens einer Oberfläche des Polyurethanschaums (902) angeordnet ist, und wobei der Film (904) eine Dicke FT von etwa 0,0076 mm (0,0003 Zoll) bis etwa 0,254 mm (0,010 Zoll) aufweist und Porendurchmesser von etwa 0,0127 mm (0,0005 Zoll) bis etwa 0,127 mm (0,005 Zoll) aufweist.

2. Klammeranordnung nach Anspruch 1, wobei der Film (904) Polyurethan (902) umfasst, das eine höhere Dichte als der Polyurethanschaum (902) aufweist.

3. Klammeranordnung nach Anspruch 1, wobei der Film (904) auf mindestens eine Oberfläche des Polyurethanschaums laminiert ist und ein absorbierbares Material umfasst, das aus der Gruppe ausgewählt ist, bestehend aus Polydioxanon (PDO), Poly(delta-gluconolacton) (PGL-1), Poly(glykolid/I-lactid) (PGL-2), Polyglykolsäure (PGA), einem Glykolid- und Epsilon-Caprolacton-Copolymer (PGCL), einem Glycolid- und I-Lactid-Copolymer, Urethan, Polycaprolacton (PCL), Polyglactin 370 (PG-370), Polyglactin 185 (PG-185) oder Kombinationen davon.

4. Klammeranordnung nach Anspruch 3, wobei der Film (904) eine Dicke FT von etwa 0,0076 mm (0,0003 Zoll) bis etwa 0,076 mm (0,003 Zoll) aufweist.

5. Klammeranordnung nach Anspruch 3 oder 4, wobei der Film (904) mit einem Klebstoff an mindestens einer Oberfläche des Polyurethanschaums befestigt ist.

6. Klammeranordnung nach Anspruch 3, wobei das absorbierbare Material Polyglactin 370 umfasst.

7. Klammeranordnung nach Anspruch 1, wobei:
das Deck (206) eine Oberfläche und mindestens einen Pfosten (1104) umfasst, der sich von der Oberfläche weg erstreckt, wobei der mindestens eine Pfosten (1104) eine Oberseite (1106) mit einem ersten Durchmesser PDT und eine Unterseite (1108) mit einem zweiten Durchmesser PDB aufweist, der kleiner als der erste Durchmesser PDT ist,
der Film (904) auf der Oberfläche angeordnet ist und den mindestens einen Pfosten (1104) mindestens teilweise umgibt, wobei zwischen der Oberseite (1106) des mindestens einen Pfostens (1104) und dem Film (904) ein Spalt (1110) besteht; und
das chirurgische Hilfsmittel (604) auf dem Film (904) angeordnet ist und den mindestens einen Pfosten mindestens teilweise umgibt.

8. Klammeranordnung nach Anspruch 7, wobei der Film (904) ein perforiertes Muster umfasst, das einen oder mehrere Klammerschlitze (1112) umgibt.

9. Verfahren zum Herstellen eines chirurgischen Werkzeugs (200), umfassend:
Bereitstellen eines chirurgischen Hilfsmittels (604), umfassend Polyurethanschaum (902), umfassend ein Volumenverhältnis des Polyurethanschaums (902) zu dem Gesamtvolumen des chirurgischen Hilfsmittels (604) in einem Bereich von etwa 0,125 bis etwa 0,325; und
Anordnen oder Ausbilden eines Films (904) auf dem Polyurethanschaum (902), wobei der Film (904) eine Dicke FT von etwa 0,0076 mm (0,0003 Zoll) bis etwa 0,254 mm (0,010 Zoll) aufweist und Porendurchmesser von etwa 0,0127 mm (0,0005 Zoll) bis etwa 0,127 mm (0,005 Zoll) aufweist.

10. Verfahren nach Anspruch 9, umfassend:
Anordnen mindestens eines flüchtigen Lösungsmittels (906) auf mindestens einem Abschnitt mindestens einer Oberfläche des Polyurethanschaums (902);
Anordnen des Films (904) auf dem mindestens einen flüchtigen Lösungsmittel (906); und
Auflösen mindestens eines Abschnitts des Polyurethanschaums (902) und des Films (904) mit dem mindestens einen flüchtigen Lösungsmittel, um den Polyurethanschaum (902) und den Film (904) zu verbinden.

11. Verfahren nach Anspruch 9, umfassend:
Bereitstellen des Polyurethanschaums (902);
Anordnen eines reaktiven Klebstoffs (906) auf mindestens einem Abschnitt des Films (904), mindestens einem Abschnitt des Polyurethanschaums (902) oder beidem; und
derartiges Anordnen des Films auf dem mindestens Polyurethanschaum (902), dass der reaktive Klebstoff (906) zwischen dem Polyurethanschaum (902) und dem Film (904) sandwichartig eingeschlossen ist.

## Revendications

1. Ensemble d'agrafage (600), comprenant :
un plateau (206) ; et
un auxiliaire chirurgical (604) disposé sur le plateau (206) et comprenant une mousse de polyuréthane (902) avec un rapport volumétrique de la mousse de polyuréthane (902) au volume total de l'auxiliaire chirurgical (604) entre environ 0,125 et environ 0,325, dans lequel l'auxiliaire chirurgical (604) comprend un film (904) disposé sur au moins une surface de la mousse de polyuréthane (902), et dans lequel le film (904) a une épaisseur FT d'environ 0,0076 mm (0,0003 pouce) à environ 0,254 mm (0,010 pouce) et a des diamètres de pore d'environ 0,0127 mm (0,0005 pouce) à environ 0,127 mm (0,005 pouce).

2. Ensemble d'agrafage selon la revendication 1, dans lequel le film (904) comprend du polyuréthane (902) ayant une densité plus élevée que la mousse de polyuréthane (902).

3. Ensemble d'agrafage selon la revendication 1, dans lequel le film (904) est laminé sur l'au moins une surface de la mousse de polyuréthane et comprend un matériau absorbable choisi dans le groupe constitué de polydioxanone (PDO), poly(delta-gluconolactone) (PGL-1), poly(glycolide/l-lactide) (PGL-2), acide polyglycolique (PGA), copolymère de glycolide et d'epsilon caprolactone (PGCL), copolymère de glycolide et de l-lactide, uréthane, poly caprolactone (PCL), polyglactine 370 (PG-370), polyglactine 185 (PG-185), ou des combinaisons de ceux-ci.

4. Ensemble d'agrafage selon la revendication 3, dans lequel le film (904) a une épaisseur FT d'environ 0,0076 mm (0,0003 pouce) à environ 0,076 mm (0,003 pouce).

5. Ensemble d'agrafage selon la revendication 3 ou 4, dans lequel le film (904) est fixé à l'au moins une surface de la mousse de polyuréthane avec un adhésif.

6. Ensemble d'agrafage selon la revendication 3, dans lequel le matériau absorbable comprend du polyglactine 370.

7. Ensemble d'agrafage selon la revendication 1, dans lequel :
le plateau (206) comprend une surface et au moins un montant (1104) s'étendant loin de la surface, l'au moins un montant (1104) ayant un sommet (1106) avec un premier diamètre PDT et un fond (1108) avec un second diamètre PDB qui est inférieur au premier diamètre PDT,
le film (904) est disposé sur la surface et entoure au moins partiellement l'au moins un montant (1104) avec un espace (1110) entre le sommet (1106) de l'au moins un montant (1104) et le film (904) ; et
l'auxiliaire chirurgical (604) est disposé sur le film (904) et entoure au moins partiellement l'au moins un montant.

8. Ensemble d'agrafage selon la revendication 7, dans lequel le film (904) comprend un motif perforé entourant une ou plusieurs fentes d'agrafes (1112).

9. Procédé de fabrication d'un outil chirurgical (200), comprenant :
la fourniture d'un auxiliaire chirurgical (604), comprenant une mousse de polyuréthane (902) dont un rapport volumétrique de la mousse de polyuréthane (902) au volume total de l'auxiliaire chirurgical (604) est compris entre environ 0,125 et environ 0,325 ; et
la disposition ou la formation d'un film (904) sur la mousse de polyuréthane (902), dans lequel le film (904) a une épaisseur FT d'environ 0,0076 mm (0,0003 pouce) à environ 0,254 mm (0,010 pouce) et a des diamètres de pore d'environ 0,0127 mm (0,0005 pouce) à environ 0,127 mm (0,005 pouce).

10. Procédé selon la revendication 9, comprenant :
la disposition d'au moins un solvant volatil (906) sur au moins une partie d'au moins une surface de la mousse de polyuréthane (902) ;
la disposition du film (904) sur l'au moins un solvant volatil (906) ; et
la dissolution d'au moins une partie de la mousse de polyuréthane (902) et du film (904) avec l'au moins un solvant volatil pour joindre la mousse de polyuréthane (902) et le film (904).

11. Procédé selon la revendication 9, comprenant :
la fourniture de la mousse de polyuréthane (902) ;
la disposition d'un adhésif réactif (906) sur au moins une partie du film (904), au moins une partie de la mousse de polyuréthane (902), ou les deux ; et
la disposition du film sur l'au moins une mousse de polyuréthane (902) de manière à ce que l'adhésif réactif (906) soit pris en sandwich entre la mousse de polyuréthane (902) et le film (904).
